# EUROPEAN PATENT SPECIFICATION

(11) **EP 1 216 704 B1**
(45) Date of publication and mention of the grant of the patent: **08.09.2004**
(21) Application number: 01310755.2
(22) Date of filing: 21.12.2001
(51) Int. Cl.: A61K 31/19, A61K 9/20, A61P 25/08, A61P 25/06, A61P 25/18

(54) **Controlled release valproate composition**
Valproat-Formulierungen mit gesteuerter Freigabe
Preparation a liberation controlee de valproate

(30) Priority: 22.12.2000 US 748566; 08.06.2001 US 877682
(43) Date of publication of application: 26.06.2002
(73) Proprietor: Abbott Laboratories, Abbott Park, Illinois 60064 (US)
(72) Inventor: Qiu, Yihong, Gumee, Illinois 60031 (US); Bollinger, Daniel J., Libertyville, IL 60048 (US); Dutta, Sandeep, Waukegan, IL 60085 (US); Cheskin, Howard S., Glencoe, IL 60022 (US); Engh, Kevin R., Kenosha, WI 53143 (US); Poska, Richard P., Mundelein, IL 60060 (US); Sommerville, Kenneth W., Libertyville, IL 60048 (US)
(74) Representative: Wright, Robert Gordon McRae

(56) References cited:
- WO-A-00/37055
- WO-A-00/45793
- WO-A-94/27587
- US-A- 4 369 172
- US-A- 4 699 927
- US-A- 5 019 398
- US-A- 6 150 410

## Description

The present invention relates to an oral controlled release composition, including a valproate compound, suitable for once-a-day administration and their use in the treatment of bipolar disorder and migraine.

More particularly, the present invention concerns a composition comprising valproic acid, a pharmaceutically acceptable salt, ester, or amide thereof, or divalproex sodium, in a controlled release composition. These controlled release compositions have an improved pharmacokinetic profile compared to known valproate compositions, in particular commercially available delayed release valproate compositions, such as Depakote DR. These compositions minimize the variance between peak and trough plasma levels of valproate, resulting in a reduction in the incidence of side effects. These compositions may be used in the treatment of bipolar disorders or migraine.

2-Propylpentanoic acid, more commonly known as valproic acid ("VPA") is effective as an antiepilpetic agent. After ingestion, the free acid dissociates to the valproate ion within the gastrointestinal tract. The valproate ion is absorbed and produces the therapeutic effect described above.

Divalproex sodium is effective in the treatment of epilepsy, migraine, and bipolar disorders. It also dissociates to the valproate ion within the gastrointestinal tract. This substance is described in more detail in United States Patent No. 4,988,731, and United States Patent No. 5,212,326.

The acid moiety of valproic acid has been functionalized in order to produce prodrugs capable of generating a valproate ion in-vivo. For example, the amide of valproic acid , valpromide ("VPO"), has been produced, as well certain salts and esters of the acid.

Despite the efficacy of these drugs in the treatment of conditions such as epilepsy, they all suffer from a common disadvantage. These valproate compounds have a relatively short half life. For example, the half life of valproic acid is reported to be between six and seventeen hours in adults and between four and fourteen hours in children. This leads to substantial fluctuations in the plasma concentration of the drug, especially in chronic administration. To maintain reasonably stable plasma concentrations, it is necessary to resort to frequent dosing, and the resulting inconvenience to the patient often results in lowered compliance with the prescribed dosing regimen. Moreover, widely fluctuating plasma concentrations of the drug may result in administration of less than therapeutic amounts of the drug in a conservative dosing regimen, or amounts too large for the particular patient in an aggressive dosing regimen. The logical solution to this problem would be to develop sustained release dosage forms that decrease the dosing frequency of the compounds.

However, the pharmacokinetics of valproic acid, and other valproate compounds, has complicated such development efforts. The relationship between plasma concentration and clinical response is not well documented for valproate. One contributing factor is the nonlinear, concentration dependent protein binding of valproate, which affects the clearance of the drug. As the dose of valproate increases, serum levels rise faster than might be expected since proportionately less of the dose is bound to plasma proteins. For example, because the plasma protein binding of valproate is concentration dependant, the free fraction increases from approximately 10% at 40µg/ml to 18.5% at 130µg/ml.

These nonlinear kinetics significantly increase the difficulty of designing sustained release dosage forms. Identical doses of the valproate compound can produce vastly different blood levels depending upon the rate at which the valproate compound is released from the dosage form.

Further complicating development efforts is the fact that a correlation between valproate levels and efficacy is unknown for disease states other than epilepsy. For example, therapeutic concentrations required to treat migraine headaches and bipolar disorders have not been established.

What impact valproate levels play in a number of side effects is also unknown at the present time. GI irritation is very common in patients consuming valproate, affecting up to one third of patients. The incidence increases at elevated doses. It is unknown if this side effect is caused by local irritation within the GI tract or is mediated via the stimulation of a receptor within the central nervous system (and thus is dependant upon plasma valproate levels). Other side effects such as asthenia, dizziness, somnolence, alopecia, and weight gain are quite common. It is also unknown if these side effects can be correlated with plasma levels of valproate. A more detailed discussion of valproate side effects may be found in PDR supra, page 421-437

In spite of the nonlinear kinetics of the compounds, a concerted effort has been devoted to the discovery of valproate compositions that will maintain more constant plasma levels of the drug following administration. The ultimate goal of these studies has been the discovery of a composition which affords stable plasma levels in a once-a-day dosing regimen. These efforts fall generally into one of two categories: (a) finding a form of the active ingredient which is more slowly released to the body metabolically, and (b) finding a composition which delivers the drug by either a timed- or controlled-release mechanism.

United States Patent No. 4,369,172 to Schor, *et al.* describes, for example, a prolonged release therapeutic composition based on mixtures of hydroxypropyl methylcellulose, ethyl cellulose and/or sodium carboxymethyl cellulose. The patentees provide a long list of therapeutic agents which they suggest can be incorporated into the composition including sodium valproate.

United States Patent No. 4,913,906 to Friedman, *et al.* discloses a controlled release dosage form of valproic acid, its amide, or one of its salts or esters in combination with a natural or synthetic polymer, pressed into a tablet under high pressure.

United States Patent No. 5,009,897 to Brinker, *et al.* discloses granules, suitable for pressing into tablets, the granules comprising a core of divalproex sodium and a coating of a mixture of a polymer and microcrystalline cellulose.

United States Patent No. 5,019,398 to Daste discloses a sustained-release tablet of divalproex sodium in a matrix of hydroxypropyl methylcellulose and hydrated silica.

United States Patent No. 5,055,306 to Barry, *et al.* discloses an effervescent or water-dispersible granular sustained release composition suitable for use with a variety of therapeutic agents. The granules comprise a core comprising the active ingredient and at least one excipient, and a water insoluble, water-swellable coating comprising a copolymer of ethyl acrylate and methyl methacrylate and a water soluble hydroxylated cellulose derivative. The patentees suggest a list of therapeutic agents which may be used in the composition of the invention, including sodium valproate.

United States Patent No. 5,169,642 to Brinkler, *et al*. discloses a sustained release dosage form comprising granules of divalproex sodium or amides or esters of valproic acid coated with a sustained release composition comprising ethyl cellulose or a methacrylic methyl ester, a plasticizer, a detackifying agent, and a slow-release polymeric viscosity agent.

United States Patent No. 5,185,159 to Aubert, *et al.* discloses a composition of valproic acid and sodium valproate which is prepared without the use of either a binder or a granulating solvent. The composition optionally contains precipitated silica as an anti-sticking or detackifying agent.

United States Patent No. 5,589,191 to Exigua, *et al*. discloses a slow release sodium valproate tablet composition in which the tablets are coated with ethyl cellulose containing silicic acid anhydride.

Published PCT application WO 94/27587 to Ayer, *et al*. discloses a method for control of epilepsy by delivering a therapeutic composition of divalproex sodium in combination with a poly (alkylene oxide).

Bialer, *et al*., "Metabolism of Antiepileptic Drugs," pp. 143-151, R. H. Levy, Ed., Raven Press, New York, 1984; Int. J. Pharmaceutics, 20: 53-63 (1984); and Biopharmaceutics and Drug Disposition, 6: 401-411 (1985); and Israel J. Med. Sci., 20: 46-49 (1995) report the pharmacokinetic evaluation of several sustained release compositions of valproic acid.

Despite all of these efforts, there remains the need for a sustained release composition of divaproex sodium, and other valproate compounds, that will permit once-a-day dosing. Further, there remains the need for a composition which will effectively maintain plasma concentrations of the drug at more constant levels over a 24 hour dosing period (i.e. minimize the variation between peak and trough plasma levels). Further, sustained release compositions are needed that will decrease the incidence of side effects associated with valproate therapy. More specifically, there remains the need to reduce the incidence of nausea, vomiting, asthenia, somnolence, alopecia, weight gain, etc. in patients undergoing valproate therapy.

WO 00/37055 discloses a controlled release formulation of divalproex sodium and it use in the treatment of epilepsy.

We have now found that compositions wih the same release characteristics as the formulation described in this application are effective in the treatment of migraine and bipolar disorder.

Use of divaloproex sodium in the manufacture of a medicament for the treatment of migraine or bipolar disorder, the medicament comprising an oral controlled release composition suitable for once-a-day administration said composition on oral ingestion producing:
i) a Cₘₐₓ that is statistically significantly(p<0.05) lower than the Cₘₐₓ produced by an equal total daily dose of delayed release divalproex sodium tablet, when each is determined at steady state in a fasting population,
ii) a Cₘᵢₙ that is not statistically significantly (p<0.05) higher than the Cₘᵢₙ produced by said equal total daily dose of delayed release divalproex sodium tablet, when each Cₘᵢₙ is determined at steady state in a fasting population,
iii) an AUC value that is equivalent to the AUC value generated by said equal total daily dose of divalproex sodium delayed release tablet, when each AUC is determined at steady state in a fasting population,
in which said delayed release divalproex sodium has a tablet core comprising, (w/w):

| | |
|---|---|
| Divalproex sodium | 62.7% |
| Polyvinylpyrrolidone | 5.8% |
| Pregelatinised starch | 11.7% |
| Silicon dioxide | 19.8% |

and a coating comprising (%of core tablet weight)

| (a) subcoat | |
|---|---|
| Polyvinylpyrrolidone | 1.9% |
| Talc | 3.2% |
| Titanium dioxide | 0.6% |

| (b) enteric coat | |
|---|---|
| Hypromellose phthalate | 5% |
| Diacetylated monoglycerides | 0.5% |
| Dyes | 0.033% |
| Titanium dioxide | 0.35%. |

The compositions of the invention exhibit significant advantages over the sustained release valproate compositions of the prior art. This composition minimizes the variation between peak and trough plasma levels of valproate over a 24 hour dosing period. This composition follows a zero-order release pattern thus producing essentially flat plasma levels of valproate, once steady-state levels have been achieved. This results in a significantly lower incidence of side effects for patients consuming such a composition, when compared to an equal total daily dose of a bid or tid composition.

Peak concentrations of valproate, Cₘₐₓ, are statistically significantly (p<0.05) below those produced by valproate dosage forms suitable for twice a day administration when measured over a 24 hour period. Trough levels of valproate, Cₘᵢₙ, are not statistically significantly different from those obtained with a twice-a-day dosage form (over 24 hours). The extent of absorption, as defined by area under the curve ("AUC"), is equivalent to those produced by the twice-a-day valproate dosage forms (over 24 hours). Such a combination of properties has unexpected benefits. It allows therapeutic levels of valproate to be maintained over a 24 hour dosing period. Further, it has been discovered that a significantly lower incidence of side effects has been achieved by this reduction in peak plasma concentration. Gastrointestinal side effects, alopecia, and certain CNS side effects have been reduced.

The once-a-day composition ("qd") comprises a valproate compound that is in association with at least one pharmaceutically acceptable polymer. A sufficient quantity of the polymer is utilized, so that upon ingestion, steady state plasma valproate levels are obtained having a degree of fluctuation that is lower than that produced by a corresponding twice-a-day valproate dosage form. The qd composition also typically provides for total absorption (AUC) of the valproate compound that is at least 80% of that achieved by a daily dose of the corresponding twice-a-day composition.

It is important to emphasize that the compositions of this invention are not limited to any one particular mechanism of drug release. Given the guidance of this patent application, one skilled in the art could achieve the enhanced pharmacokinetic and side effect profile using any oral controlled release polymeric dosage form known in the art. This includes osmotic pump systems, matrix systems, or reservoir systems.

A more specific embodiment of this invention is directed to a once-a-day divalproex sodium dosage form. This composition has a degree of fluctuation that is less than that achieved by a divalproex sodium delayed release tablet. This qd dosage form also produces total valproate absorption that is at least 80% of that achieved by the divalproex sodium delayed release tablets. Peak steady state serum valproate levels obtained with the qd dosage form are 10-20 % lower than that produced by the divalproex sodium delayed release tablets. Trough levels, which are important in maintaining control of epileptic seizures, are not statistically significantly different from those obtained with the divalproex sodium delayed release tablets following administration of equal doses of the once-a-day and delayed-release compositions.

### I. Definitions

As noted above, the invention relates to new and improved compositions of valproate compounds which disassociate *in-vivo* to produce a valproate ion. Several valproate compounds are currently available commercially in the United States or have been described in the literature.

One such compound is valproic acid. Valproic acid may be represented by the following structure:

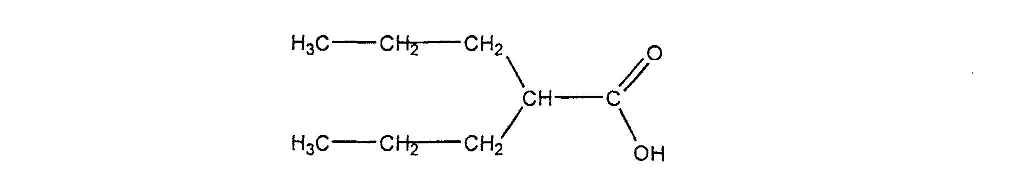

Valproic acid is available commercially from Abbott Laboratories of Abbott Park, Illinois. Methods for its synthesis are described in Oberreit, Ber. 29, 1998 (1896) and Keil, Z. Physiol. Chem. 282, 137 (1947). Its activity as an antiepileptic compound is described in the PDR, 52nd Edition, page 421, 1998. Upon oral ingestion within the gastrointestinal tract, the acid moiety disassociates to form a carboxylate moiety (i.e. a valproate ion).

The sodium salt of valproic acid is also known in the art as an anti-epileptic agent. It is also known as sodium valproate and is described in detail in The Merck Index, 12 Edition, page 1691, (1996). Further descriptions may be found in the PDR, 52^{nd} Edition, page 417, (1998).

Divalproex sodium is effective as an antiepileptic agent and is also used for migraine and bipolar disorders. Methods for its preparation may be found in United States Patent No.'s 4,988,731 and 5,212,326. Like valproic acid, it also disassociates within the gastrointestinal tract to form a valproate ion.

As used in this application:
a) "Cₘₐₓ" means maximum plasma concentration of the valproate ion, produced by the ingestion of the composition of the invention or the twice-a-day comparator (BID).
b) "Cₘᵢₙ" means minimum plasma concentration of the valproate ion, produced by the ingestion of the composition of the invention or the BID comparator.
c) "C_{avg}" means the average concentration of valproate ion within the 24-hour interval produced by the ingestion of the composition of the invention or the BID comparator. C_{avg} is calculated as AUC over a 24 hour interval divided by 24.
d) "Tₘₐₓ" means time to the maximum observed plasma concentration produced by the ingestion of the composition of the invention or the BID comparator.
e) "AUC" as used herein, means area under the plasma concentration-time curve, as calculated by the trapezoidal rule over the complete 24-hour interval for all the compositions.
g) "Degree of Fluctuation (DFL)" as used herein, is expressed as: DFL=(Cₘₐₓ-Cₘᵢₙ)/C_{avg} produced by the ingestion of the composition of the invention or the BID comparator.
g) "Pharmaceutically acceptable" as used herein, means those salts/polymers/excipients which are, within the scope of sound medical judgment, suitable for use in contact with the tissues of humans and lower animals without undue toxicity, irritation, allergic response, and the like, in keeping with a reasonable benefit/risk ratio, and effective for their intended use in the treatment and prophylaxis of migraine, bipolar disorders,
h) "Side effects" as used herein, means those physiological effects to various systems in the body such as cardiovascular systems, nervous system, digestive system, and body as a whole, which cause pain and discomfort to the individual subject, and which are the direct result of the ingestion of the valproate compound.
i) "decreased incidence of side effects" refers to a reduced incidence of side effects in a patient population, and not to a total absence of side effects, when measured in a comparable population consuming a valproate dosage form suitable for twice daily administration. As is well known to those skilled in the art, even placebo dosage forms made of sugar produce some measurable incidence of side effects. Thus an improved side effect profile must be interpreted in light of the relevant art.
j) " delayed release divalproex sodium tablets" refers to an enteric coated dosage form containing divalproex sodium intended to delay the release of the medication until the dosage form has passed through the stomach.
k) "bid" refers to the administration of a composition twice during a 24 hour period.
l) "qd" refers to a dosage form that may be administered once during a 24 hour period.
m) A statistical test is said to be statistically significant where the resulting p-value is less than or equal to 0.05, unless otherwise noted. Equivalence and statistical significance are not synonymous.

As used in this application, the terms "Cₘᵢₙ" and "trough levels", should be considered synonyms. Likewise, the terms "Cₘₐₓ" and "peak levels" should also be considered synonyms. Any reference to a plasma concentration of valproate ion, and more specifically to any quantification thereof, such as, for example, Cₘᵢₙ, Cₘₐₓ, AUC, DFL, etc., should be considered to have been determined at steady state in a fasting population, unless expressly stated otherwise. Any reference in the specification or claims to a comparison of pharmacokinetic values (i.e. Cₘₐₓ, Cₘᵢₙ, AUC, DFL, etc.) or, of side effects, between a bid composition of the prior art and a qd composition of this invention, should be considered to be based upon identical total daily doses of the valproate compound, unless expressly stated otherwise. Any reference in the specification or claims to a pharmacokinetic value in a patient population should be construed as referring to a group of subjects and not an individual.

### II. Pharmacokinetic Profile

As noted above, the invention resides in the discovery that a composition having an improved pharmacokinetic profile will simultaneously accomplish two results. First, it will provide a dosage form of valproate that will maintain therapeutic levels of the valproate ion over a 24 hour dosing period, thus providing once daily dosing. Secondly, it will reduce the incidence of side effects associated with valproate therapy.

In order to obtain these benefits, it is necessary for the once-a-day valproate dosage form to achieve certain pharmacokinetic parameters, when compared to a bid valproate dosage form. The qd dosage form must reduce peak plasma levels of valproate (Cₘₐₓ) without significantly impacting either trough levels (Cₘᵢₙ) or the extent of valproate absorption (AUC). Further, the qd dosage form will exhibit a DFL that is lower than that exhibited by a corresponding bid valproate dosage form.

Cₘₐₓ for the qd dosage form should be statistically significantly lower than the Cₘₐₓ for a bid dosage form of the same valproate compound, when each is measured at steady state in a fasting population. For example, a once-a-day divalproex sodium dosage form will exhibit a Cₘₐₓ that is statistically significantly lower than that produced by a divalproex sodium delayed release tablet, when each is measured at steady state in a fasting population. Typically, peak plasma levels of valproate are reduced at least 10%. More typically, these peak plasma levels are reduced up to about 20%. This reduction must be accomplished with out any significant reduction in trough levels or total absorption of valproate.

Cₘᵢₙ for the qd dosage form should not be statistically significantly different from that obtained with a bid dosage form of the same valproate compound, when each is determined at steady state in a fasting population. More specifically, Cₘᵢₙ for a once-day divalproex sodium dosage form should not be statistically significantly different from that obtained with a delayed release divalproex sodium tablet when each is measured at steady state in a fasting population. Maintaining comparable trough levels to those obtained with the prior art bid dosage forms is necessary to maintain the therapeutic efficacy of the valproate compound. Inadequate trough levels are associated with seizures in epileptic patients.

In addition to reducing peak valproate levels as described above, it is also important that the total amount of valproate absorbed from the qd dosage form not be decreased significantly, when compared to a bid dosage form of the same valproate compound when dosed over a 24 hour dosing interval. Total drug absorption is also referred to as AUC (area under the curve). Methods for quantifying drug absorption are well known to those skilled in the art and have been standardized by the United States Food and Drug Administration at www.fda.gov/cder/guidance/stat-two.pdf.

AUC for the qd dosage form will be equivalent to the AUC of the bid dosage form of the same valproate compound when each is measured at steady state in a fasting population over a 24 hour period. Equivalence of a pharmacokinetic parameter refers to the 90% confidence interval of the ratio of the central values of the pharmacokinetic parameter of the test composition to the reference composition being contained within 0.80 to 1.25. More specifically, the AUC of qd divalproex sodium tablet form will be equivalent to that obtained with a delayed release divalproex sodium dosage form when each is determined at steady state in a fasting population over a 24 hour dosing period.

An AUC of at least 80% should be achieved with the compositions used in this invention, when compared to a bid dosage form over a 24 hour interval. Values below 80% tend to negatively impact trough levels leading to sub-therapeutic concentrations of valproate and loss of epileptic control, etc. AUC's in excess of 125% should also be avoided. Thus with respect to the extent of absorption, the compositions of this invention should be considered equivalent to the corresponding bid valproate dosage form.

Degree of Fluctuation ("DFL") is a measurement of how much plasma levels of a drug vary over the course of a dosing interval. The closer the DFL is to zero (o), the less variance there is over the course of a dosing period. Thus a reduced DFL signifies that the difference in peak and trough plasma levels has been reduced. The DFL for a qd dosage form of this invention will be lower than that of the corresponding bid dosage form, for the same valproate compound, when each is evaluated at steady state in a fasting population. In a more specific embodiment, a qd divalproex sodium dosage form will have a DFL that is lower than that achieved with a bid delayed release divalproex sodium tablet when each is evaluated at steady state in a fasting population.

Despite the numerous therapeutic advantages of valproate therapy, certain patients consuming these medications experience side effects. For example, with divalproex sodium delayed release tablets, approximately 7% of patients report alopecia (hair loss) PDR supra, page 435-436. Up to 8% of patients report significant weight gain PDR supra, page 435-436. Such side effects can have disasterous consequences for the self image of patients, especially for females, or younger patients. It is unknown whether this hair loss or weight gain is associated with obtaining or maintaining certain plasma levels of valproate

Likewise, up to one-third of patients consuming divalproex sodium delayed release tablets report suffering from nausea. While such an event is certainly not life threatening, it is unpleasant for the patient. The nausea can lead to non-compliance and subsequent worsening of the patient's disease. Dizziness, tremor, asthenia, somnolence are also common with valproate therapy. The impact of plasma levels on these side effects is also unknown. For a more complete discussion of valproate side effects, please refer to PDR supra, page 421-437.

The incidence of these side effects can be reduced significantly by reducing peak plasma levels of valproate by approximately 10-20%. Further, therapeutic control can be maintained by meeting the DFL, Cₘᵢₙ, and AUC guidelines discussed above. Such a finding was totally unexpected. The literature clearly documents that the correlation between side effects and plasma valproate levels is unknown.

### III. Dosage Forms

As noted above, the benefits of this invention are not limited to a single type of dosage form having a particular mechanism of drug release. This enhanced pharmacokinetic profile can be obtained with any of the oral sustained release dosage forms in use today, following the teachings above.

As of the filing date of this application, there are three types of commonly used oral polymeric controlled release dosage forms. This includes matrix systems, osmotic pumps, and membrane controlled technology (also referred to as reservoir systems). Each of these systems is described in greater detail below. A detailed discussion of such dosage forms may also be found in: (i) Handbook of pharmaceutical controlled release technology, ed. D. L. Wise, Marcel Dekker, Inc. New York, New York (2000), and (ii). Treatise on controlled drug delivery, fundamentals, optimization, and applications, ed. A. Kydonieus, Marcel Dekker, Inc. New York, New York (1992).

### A) Matrix Systems

Matrix systems are well known in the art. In a matrix system, the drug is homogenously dispersed in a polymer in association with conventional excipients. This admixture is typically compressed under pressure to produce a tablet. Drug is released from this tablet by diffusion and erosion. Matrix systems are described in detail by Wise and Kydonieus, supra.

The matrix compositions of this invention comprise a valproate compound and a pharmaceutically acceptable polymer. Preferably, the valproate compound is divalproex sodium. The amount of the valproate compound varies from about 40% to about 80% by weight of the dosage form. Preferably, the dosage form comprises about 45% to about 65% by weight of the valproate compound.

The pharmaceutically acceptable polymer is a water-soluble hydrophilic polymer, or a water insoluble hydrophobic polymer (including waxes). Examples of suitable water soluble polymers include polyvinylpyrrolidine, hydroxypropylcellulose, hydroxypropylmethyl cellulose, methyl cellulose, vinyl acetate copolymers, polysaccharides (such as alignate, xanthum gum, etc.), polyethylene oxide, methacrylic acid copolymers, maleic anhydride/methyl vinyl ether copolymers and derivatives and mixtures thereof. Examples of suitable water insoluble polymers include acrylates, cellulose derivatives such ethylcellulose or cellulose acetate, polyethylene, methacrylates, acrylic acid copolymers and high molecular weight polyvinylalcohols. Examples of suitable waxes include fatty acids and glycerides.

Preferably, the polymer is selected from hydroxypropyl cellulose, hydroxypropylmethyl cellulose, and methyl cellulose. More preferably, the polymer is hydroxypropylmethyl cellulose. Most preferably, the polymer is a high viscosity hydroxypropyl-methyl cellulose with viscosity ranging from about 4,000 cps to about 100,000 cps. The most preferred high viscosity polymer is a hydroxypropylmethyl cellulose with a viscosity of about 15,000 cps, commercially available under the Tradename, Methocel, from The Dow Chemical Company.

The amount of the polymer in the dosage form generally varies from about 20% to about 50% by weight of the composition. Preferably, the amount of polymers varies from about 25% to about 45% by weight of the dosage form. Most preferably, the amount of polymer varies from about 30% to about 40% by weight of the dosage form.

The composition of the invention also typically includes pharmaceutically acceptable excipients. As is well known to those skilled in the art, pharmaceutical excipients are routinely incorporated into solid dosage forms. This is done to ease the manufacturing process as well as to improve the performance of the dosage form. Common excipients include diluents or bulking agents, lubricants, binders, etc. Such excipients are routinely used in the dosage forms of this invention.

Diluents, or fillers, are added in order to increase the mass of an individual dose to a size suitable for tablet compression. Suitable diluents include powdered sugar, calcium phosphate, calcium sulfate, microcrystalline cellulose, lactose, mannitol, kaolin, sodium chloride, dry starch, sorbitol, etc.

Lubricants are incorporated into a composition for a variety of reasons. They reduce friction between the granulation and die wall during compression and ejection. This prevents the granulate from sticking to the tablet punches, facilitates its ejection from the tablet punches, etc. Examples of suitable lubricants include talc, stearic acid, vegetable oil, calcium stearate, zinc stearate, magnesium stearate, etc.

Glidant's are also typically incorporated into the composition. A glidant improves the flow characteristics of the granulation. Examples of suitable glidant's include talc, silicon dioxide, and cornstarch.

Binders may be incorporated into the composition. Binders are typically utilized if the manufacture of the dosage form uses a granulation step. Examples of suitable binders include povidone, polyvinylpyrrolidone, xanthan gum, cellulose gums such as carboxymethylcellulose, methyl cellulose, hydroxypropylmethylcellulose, hydroxycellulose, gelatin, starch, and pregelatinized starch.

Other excipients that may be incorporated into the composition include preservatives, antioxidants, or any other excipient commonly used in the pharmaceutical industry, etc.

The amount of excipients used in the composition will correspond to that typically used in a matrix system. The total amount of excipients, fillers and extenders, etc. varies from about 10% to about 40% by weight of the dosage form.

The matrix compositions are generally prepared using standard techniques well known in the art. Typically, they are prepared by dry blending the polymer, filler, valproate compound, and other excipients followed by granulating the mixture using an alcohol until proper granulation is obtained. The granulation is done by methods known in the art. The wet granules are dried in a fluid bed dryer, sifted and ground to appropriate size. Lubricating agents are mixed with the dried granulation to obtain the final composition.

The compositions of the invention can be administered orally in the form of tablets, pills, or the granulate may be loose filled into capsules. The tablets can be prepared by techniques known in the art and contain a therapeutically useful amount of the valproate compound and such excipients as are necessary to form the tablet by such techniques. Tablets and pills can additionally be prepared with enteric coatings and other release-controlling coatings for the purpose of acid protection, easing swallow ability, etc. The coating may be colored with a pharmaceutically accepted dye. The amount of dye and other excipients in the coating liquid may vary and will not impact the performance of the extended release tablets. The coating liquid generally comprises film forming polymers such as hydroxypropyl cellulose, hydroxypropylmethyl cellulose, cellulose esters or ethers (such as cellulose acetate or ethylcellulose), an acrylic polymer or a mixture of polymers. The coating solution is generally an aqueous solution or an organic solvent further comprising propylene glycol, sorbitan monoleate, sorbic acid, fillers such as titanium dioxide, a pharmaceutically acceptable dye.

A particularly preferred matrix system for the extended release of the valproate compound there from comprises: from about 50 weight percent to about 55 weight percent of a valproate compound; from about 20 weight percent to about 40 weight percent of hydroxypropyl methylcellulose; from about 5 weight percent to about 15 weight percent of lactose, from about 4 weight percent to about 6 weight percent of microcrystalline cellulose, and from about 1 weight percent to about 5 weight percent of silicon dioxide, in which said silicon dioxide has an average particle size ranging between about 1 micron and about 10 microns; and all weight percentages based upon the total weight of the dosage form.

This preferred embodiment of the invention also extends a dry granular composition suitable for compressing into a tablet dosage form, the granular composition comprising particles of a size smaller than about 1 mm and comprising from about 50 weight percent to about 55 weight percent of an active ingredient selected from the group consisting of valproic acid, a pharmaceutically acceptable salt or ester of valproic acid, divalproex sodium, and valpromide; from about 20 weight percent to about 40 weight percent of hydroxypropyl methylcellulose; from about 5 weight percent to about 15 weight percent of lactose, from about 4 weight percent to about 6 weight percent of microcrystalline cellulose, and from about i weight percent to about 5 weight percent of silicon dioxide, in which said silicon dioxide has an average particle size ranging between about 1 micron and about 10 microns; and all weight percentages based upon the total weight of the granular composition.

More specifically, a divalproex matrix may be prepared by a) dry blending a mixture of from about 50 weight percent to about 55 weight percent divalproex sodium, from about 20 weight percent to about 35 weight percent hydroxypropylmethyl cellulose, from about 5 weight percent to about 15 weight percent lactose to form a uniform mixture of the dry ingredients; b) wet granulating the dry uniform mixture from step a); c) drying and sizing the wet granules from step b) to select granules having an average size below 1 mm; d) dry blending the granules with from about 4 weight percent to about 6 weight percent microcrystalline cellulose, and from about 1 weight percent to about 5 weight percent silicon dioxide having an average particle size ranging between about 1 micron and about 10 microns; and e) compressing the blended granules of step h) under a force ranging between about 2000 lbf (about 8.9 x 10³ Newtons) and 10,000 lbf (about 4.45 x 10⁴ Newtons). In a similar manner, the microcrystalline cellulose can be dry blended in step (a) with the divalproex sodium, hydroxypropyl methylcellulose and lactose.

### B) Osmotic Pumps

In an osmotic pump system, a tablet core is encased by a semipermeable membrane having at least one orifice. The semipermeable membrane is permeable to water, but impermeable to the drug. When the system is exposed to body fluids, water will penetrate through the semipermeable membrane into the tablet core containing osmotic excipients and the active drug. Osmotic pressure increases within the dosage form and drug is released through the orifice in an attempt to equalize pressure.

In more complex pumps, the tablet core contains two internal compartments. The first compartment contains the drug. The second compartment contains a polymer which swells on contact with fluid. After ingestion, this polymer swells into the drug containing compartment at a predetermined rate and forces drug from the dosage form at that rate. Such dosage forms are often used when are zero order release profile is desired, such as in the instant invention.

Osmotic pumps are well known in the art and have been described in the literature. United States Patent No.'s 4,088,864; 4,200,098; and 5,573,776, describe osmotic pumps and methods for their manufacture. Osmotic pumps containing valproate compounds, such as divalproex sodium, have been described by Ayer et al in United States Patent No. 5,980,943. One skilled in the art, taking into account this applications teachings and those of the '864, '098, '776 and '943 patents could produce an osmotic pump matching the pharmacokinetic profile described above.

As a general guideline, the osmotic pumps of this invention are typically formed by compressing a tablet of an osmotically active drug (or an osmotically inactive drug in combination with an osmotically active agent or osmagent) and then coating the tablet with a semipermeable membrane which is permeable to an exterior aqueous-based fluid but impermeable to the passage of drug and/or osmagent. One or more delivery orifices may be drilled through the semipermeable membrane wall. Alternatively, orifice(s) through the wall may be formed in situ by incorporating leachable pore forming materials in the wall. In operation, the exterior aqueous based fluid is imbibed through the semipermeable membrane wall and contacts the drug and/or salt to form a solution or suspension of the drug. The drug solution or suspension is then pumped out through the orifice as fresh fluid is imbibed through the semipermeable membrane.

In a further preferred embodiment, the tablet contains two distinct compartments. The first compartment contains the drug as described above. The second compartment contains an expandable driving member consisting of a layer of a swellable hydrophilic polymer, which operates to diminish the volume occupied by the drug, thereby delivering the drug from the device at a controlled rate over an extended period of time.

Typical materials for the semipermeable membrane include semipermeable polymers known to the art as osmosis and reverse osmosis membranes, such as cellulose acylate, cellulose diacylate, cellulose triacylate, cellulose acetate, cellulose diacetate, cellulose triacetate, agar acetate, amylose triacetate, beta glucan acetate, acetaldehyde dimethyl acetate, cellulose acetate ethyl carbamate, polyamides, polyurethanes, sulfonated polystyrenes, cellulose acetate phthalate, cellulose acetate methyl carbamate, cellulose acetate succinate, cellulose acetate dimethyl aminoacetate, cellulose acetate ethyl carbamate, cellulose acetate chloracetate, cellulose dipalmitate, cellulose dioctanoate, cellulose dicaprylate, cellulose dipentanlate, cellulose acetate valerate, cellulose acetate succinate, cellulose propionate succinate, methyl cellulose, cellulose acetate p-toluene sulfonate, cellulose acetate butyrate, cross-linked selectively semipermeable polymers formed by the coprecipitation of a polyanion and a polycation as disclosed in United States Patent Nos. 3,173,876; 3,276,586; 3,541,005; 3,541,006; and 3,546,142, semipermeable polymers as disclosed by Loeb and Sourirajan in United States Patent No. 3,133,132, lightly cross-linked polystyrene derivatives, cross-linked poly(sodium styrene sulfonate), poly(vinylbenzyltrimethyl ammonium chloride), cellulose acetate having a degree of substitution up to 1 and an acetyl content up to 50%, cellulose diacetate having a degree of substitution of 1 to 2 and an acetyl content of 21 to 35%, cellulose triacetate having a degree of substitution of 2 to 3 and an acetyl content of 35 to 44.8%, as disclosed in United States Patent No. 4,160,020.

The osmotic agent present in the pump, which may be used when the drug itself is not osmotically active, are osmotically effective compounds soluble in the fluid that enters the device, and exhibits an osmotic pressure gradient across the semipermeable wall against the exterior fluid. Osmotically effective osmagents useful for the present purpose include magnesium sulfate, calcium sulfate, magnesium chloride, sodium chloride, lithium chloride, potassium sulfate, sodium carbonate, sodium sulfite, lithium sulfate, potassium chloride, sodium sulfate, d-mannitol, urea, sorbitol, inositol, raffinose, sucrose, glucose, hydrophilic polymers such as cellulose polymers, mixtures thereof, and the like. The osmagent is usually present in an excess amount, and it can be in any physical form, such as particle, powder, granule, and the like. The osmotic pressure in atmospheres of the osmagents suitable for the invention will be greater than zero and generally up to about 500 atm, or higher.

The expandable driving member is typically a swellable, hydrophilic polymer which interacts with water and aqueous biological fluids and swells or expands to an equilibrium state. The polymers exhibit the ability to swell in water and retain a significant portion of the imbibed water within the polymer structure. The polymers swell or expand to a very high degree, usually exhibiting a 2 to 50 fold volume increase. The polymers can be noncross-linked or cross-linked. The swellable, hydrophilic polymers are in one presently preferred embodiment lightly cross-linked, such cross-links being formed by covalent ionic bonds or hydrogen bonds. The polymers can be of plant, animal or synthetic origin. Hydrophilic polymers suitable for the present purpose include poly(hydroxy alkyl methacrylate) having a molecular weight of from 30,000 to 5,000,000; kappa carrageenan, polyvinylpyrrolidone having molecular weight of from 10,000 to 360,000; anionic and cationic hydrogels; polyelectrolyte complexes; poly(vinyl alcohol) having a low acetate residual, cross-linked with glyoxal, formaldehyde, or glutaraldehyde and having a degree of polymerization from 200 to 30,000; a mixture of methyl cellulose; cross-linked agar and carboxymethyl cellulose; a water insoluble, water swellable copolymer produced by forming a dispersion of finely divided copolymer of maleic anhydride with styrene, ethylene, propylene, butylene or isobutylene cross-linked with from 0.001 to about 0.5 moles of saturated cross-linking agent per mole of maleic anhydride in copolymer; water swellable polymers of N-vinyl lactams, and the like.

The expression "orifice" as used herein comprises means and methods suitable for releasing the drug from the system. The expression includes one or more apertures or orifices which have been bored through the semipermeable membrane by mechanical procedures. Alternatively it may be formed by incorporating an erodible element, such as a gelatin plug, in the semipermeable membrane. In cases where the semipermeable membrane is sufficiently permeable to the passage of drug, the pores in the membrane may be sufficient to release the agent/drug in therapeutically effective amounts. In such cases, the expression "passageway" refers to the pores within the membrane wall even though no bore or other orifice has been drilled there through. A detailed description of osmotic passageways and the maximum and minimum dimensions for a passageway are disclosed in United States Patent Nos. 3,845,770 and 3,916,899.

The osmotic pumps of this invention are manufactured by standard techniques. For example, in one embodiment, the drug and other ingredients that may be housed in one area of the compartment adjacent to the passageway, are pressed into a solid possessing dimension that corresponds to the internal dimensions of the area of the compartment the agent will occupy, or the agent and other ingredients and a solvent are mixed into a solid or semisolid form by conventional methods such as ballmilling, calendaring, stirring or rollmilling, and then pressed into a preselected shape. Next, a layer of a hydrophilic polymer is placed in contact with the layer of agent in a like manner, and the two layers surrounded with a semipermeable wall. The layering of agent composition and hydrophilic polymer can be fabricated by conventional two-layer press techniques. The wall can be applied by molding, spraying or dipping the pressed shapes into a wall forming material. Another and presently preferred technique that can be use for applying the wall is the air suspension procedure. This procedure consists of suspending and tumbling the pressed agent and dry hydrophilic polymer in a current of air and a wall forming composition until the wall is applied to the agent-hydrophilic polymer composite. The air suspension procedure is described in United States Patent No. 2,799,241; J. Am. Pharm. Assoc., Vol. 48, pp. 451-459, (1979). Other standard manufacturing procedures are described in Modern Plastics Encyclopedia, Vol. 46, pp. 62-70 (1969); and in Pharmaceutical Sciences, by Remington, Fourteenth Edition, pp. 1626-1678 (1970), published by Mack Publishing Company, Easton, PA.

### C) Reservoir Polymeric Systems

Reservoir systems are well known in the art. This technology is also commonly referred to as microencapsulation, bead technology, or coated tablets. Small particles of the drug are encapsulated with pharmaceutically acceptable polymer. This polymer, and its relative quantity, offers a predetermined resistance to drug diffusion from the reservoir to the gastrointestinal tract. Thus drug is gradually released from the beads into the gastrointestinal tract and provides the desired sustained release of valproate compound.

These dosage forms are well known in the art. United States Patent No's. 5,286,497 and 5,737,320, describe such compositions and their methods of production. United States Patent No's. 5,354,556; 4,952,402; and 4,940,588, specifically discuss using such technology to produce sustained release dosage forms of valproate compounds such as sodium valproate. One skilled in the art, taking into account this applications teachings and those of the '556, '402, '588, '320, and the '497 patents could produce a bead or pellet based dosage form matching the pharmacokinetic profile described above.

As a general guideline however, a pellet is formed with a core of a valproate compound, optionally in association with conventional excipeints. This core is then coated with one, or more, pharmaceutically acceptable polymers. Often, the coating polymer is an admixture of a major proportion of a pharmaceutically acceptable water insoluble polymer and a minor proportion of a pharmaceutically acceptable water soluble polymer.

The central core may be prepared by a number of techniques known in the art. Typically the valproate compound is bound to an inert carrier with a conventional binding agent. The inert carrier is typically a starch or sugar sphere. Before the valproate is bound to the inert carrier, it is typically blended with conventional excipients to expedite its handling and to improve the properties of the final dosage form. These excipients are identical to those described above for the matrix systems. The quantity of these excipients can vary widely, but will be used in conventional amounts. The central core is then produced by utilizing a binding agent to attach the powdered valproate blend to the solid carrier. This can be accomplished by means known in the art for producing pharmaceutical beads. Suitable means include utilization of a conventional coating pan, an automatic coating machine, or a rotogranulator. The production of these central cores is described in more detail in Pharmaceutical Pelletization Technology, ed. I. Ghebre-Sellassie, Marcel Dekker, Inc. New York, New York (1989).

The second major component of the beads is the polymeric coating. As noted above, the polymeric coating is responsible for giving the beads their sustained release characteristics. The polymeric coating may be applied to the central core using methods and techniques known in the art. Examples of suitable coating devices include fluid bed coaters, pan coaters, etc. The application techniques are described in more detail in: i) Aqueous polymeric coatings for pharmaceutical dosage forms, ed. J. W. McGinity, Marcel Dekker, Inc. New York, New York (1997); and 2) Pharmaceutical Dosage Forms: Tablets Vol. 3. ed. H. A. Lieberman, L. Lachman and J. B. Schwartz, Marcel Dekker, Inc. New York, New York pp. 77-287, (1990)..

Examples of suitable polymers include ethylcellulose, cellulose acetate, cellulose propionate (lower, medium or higher molecular weight), cellulose acetate propionate, cellulose acetate butyrate, cellulose acetate phthalate, cellulose triacetate, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), poly(ethylene), poly(ethylene) low density, poly(ethylene) high density, poly(propylene), poly(ethylene oxide), poly(ethylene terephthalate), poly(vinyl isobutyl ether), poly(vinyl acetate), poly(vinyl chloride) or polyurethane or mixtures thereof.

Once the beads have been prepared, they may be filled into capsules as is known in the art. Alternately, they may be pressed into tablets using techniques conventional in the art.

The following examples are presented in order to further illustrate the invention. While all of the examples specifically relate to matrix dosage forms, their relevance extends to any of the dosage forms described above. One skilled in the art could use their teachings to prepare reservoir systems or osmotic pumps having the pharmacokinetic profile described above.

In migraine and bipolar disorder, the individual dose of the qd composition will depend upon the patient; taking into account the severity of their illness, their age, other underlying diseases or conditions (ie. renal failure), their body weight, other drugs they are taking that may potentially impact the rate at which the valprote compound is metabolized or excreted from the body, etc. The dose of the qd composition will typically be equal to the total daily dose of valproate that they would receive from a bid dosage form. For example, a patient consuming 500mg of divalproex sodium delayed release tablets bid would be initiated on 1000mg of divalproex sodium given once daily.

### Brief Description of the Drawings

In the drawings, which form a part of this specification:
FIGURE is a graphical representation of plasma valproate levels of two qd (once-a-day) and one bid (twice-a-day) dosage form.

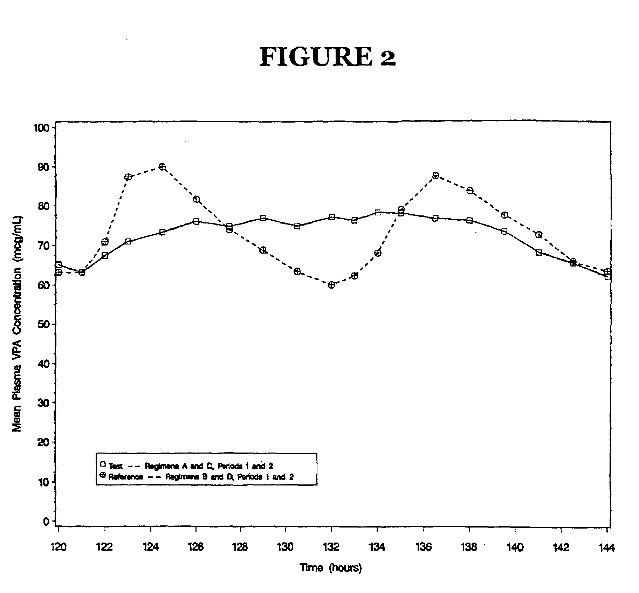

FIGURE 2 is a graphical representation of plasma valproate levels of a qd (once-a-day) and bid (twice-a-day) dosage form.

### EXAMPLES

### Example 1

This Example illustrates the manufacture of a preferred dosage form of the present invention at a large scale.

Divalproex sodium was milled through a 0.040" band with impact forward (flat edge) using a Fluid Air Mill operating at 50-75 rpm feed rate and 3500 rpm mill speed. 81 kg of milled drug was vacuum loaded directly into the Collette Gral-600 high shear mixer and mixed with 12.3 kg of lactose, 7.5 kg of microcrystalline cellulose and 45 kg of hydroxypropylmethycellulos for 5 minutes. The mixture of drug and excipients was granulated using 18 kg of purified water for a total of 7 minutes and dried in a fluid bed dryer until the average moisture content of the granules, measured by a gravimetric test, is below the in-process control limit of 1.0% w/w. The dried granules are sized using a speed sifter and the oversize granules are milled through a 0.078" band with impact forward (flat edge) using a Fluid Air Mill operating at 50 rpm feed rate and 3500 rpm mill rate. The two fractions of granules are then recombined and blended with 4.5 kg of silicon dioxide in a twin-shell blender. The blended mixture is compressed into 1.00 gram tablets with approximately 0-12 kN precompression and 24 kN main compression force using a rotary tableting machine (Fette 2090) operating at 35-50 rpm.

### Example 2

### Multiple Dose Study

The bioavailability and plasma concentration versus time profile of valproate from an oral extended-release tablet composition of divalproex sodium (made as in Example i) determined under fasting and nonfasting conditions was compared to those of a commercially available enteric coated divalproex sodium delayed-release tablet composition (Depakote®, Abbott Laboratories; reference) determined under fasting conditions in healthy subjects. The study was conducted according to a multiple-dose, open-label, three-period, randomized, complete crossover design. In each period, a six-day regimen was administered with a minimum of 16 days separating the first doses of consecutive periods. The three regimens were:
Regimen A: Extended-release composition 1000 mg q24h administered under fasting conditions (test/invention)
Regimen B: Extended-release composition 1000 mg q24h administered 30 minutes after breakfast was served (test/invention)
Regimen C: Depakote enteric coated tablet 500 mg q12h administered under fasting conditions (reference/bid comparator)

A schedule of the doses and meal times for the three regimens follows.

**Table 1**

| Regimen | Composition | Time of Dose | Breakfast | Lunch | Dinner | Snack |
|---|---|---|---|---|---|---|
| A | Test ER | 6:00 a.m. | 8:00 a.m. | 12 N | 8:00 pm | 10:30 pm |
| B | Test ER | 6:00 a.m. | 5:30 a.m. | 12 N | 8:00 pm | 10:30 pm |
| C | Reference DR | 6:00 a.m. | 8:00 a.m. | 12 N | 8:00 pm | 10:30 pm |
| | | 6:00 p.m. | | | | |
| ER = Extended-Release; DR = Delayed Release (enteric-coated). | | | | | | |

Fourteen healthy adult subjects (11 male and 3 female subjects) completed all phases of the study. The mean age was 27 years (range 19 - 51 years), mean height was 69 inches (range 63 - 74 inches) and weight was 161 pounds (range 120 - 200 pounds).

Blood samples (7 mL) were collected at o, 12, 24, 36, 48, 60, 72, 84, 96, 108, 120, 121, 122, 123, 124.5, 126, 127.5, 129, 130.5, 132, 133, 134, 135, 136.5, 138, 139.5, 141, 142.5 and 144 hours after the first dose of each period. Plasma samples were analyzed for valproic acid using a validated gas-liquid chromatographic method with flame ionization detection at Oneida Research Services, Inc., Whitesboro, New York.

### Pharmacokinetic and Statistical Analyses

Pharmacokinetic parameters were estimated by noncompartmental techniques. For Day 6 data, these included Cₘₐₓ, Tₘₐₓ, Cₘᵢₙ, AUC₀₋₂₄, and degree of fluctuation (DFL). If Cₘₐₓ for the reference occurred after the second dose of Day 6, Tₘₐₓ was taken to be the time since the second dose rather than the time from the first dose.

Analyses of variance (ANOVAs) appropriate for crossover models were performed for Tₘₐₓ, DFL, and for the natural logarithms of Cₘᵢₙ, Cₘₐₓ, and AUCₒ₋₂₄. Within the framework of the ANOVA, the regimens were compared pair-wise, each comparison done by a test at significance level of 0.05. Equivalence of the two compositions with respect to AUC was addressed by performing the two one-sided tests procedure at significance level 0.05 within the framework of the ANOVA on the logarithm of AUC. As a further aid for assessing the characteristics of the ER composition, 95% confidence intervals for the ratios of the ER composition central values to the reference regimen central value were obtained from the ANOVAs for logarithms of Cₘᵢₙ and Cₘₐₓ. In addition, a two one-sided tests procedure was carried out to compare the fasting and nonfasting extended-release composition regimens.

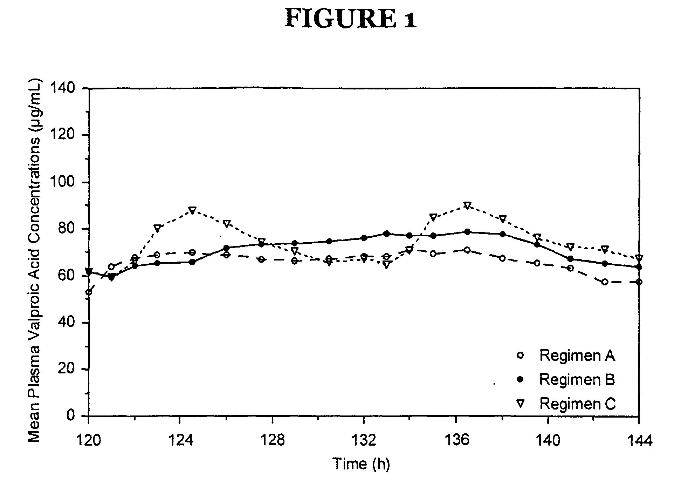

The mean valproic acid plasma concentration-time profiles for the three regimens are shown in Figure 1.

The pharmacokinetic results for Day 6 of each regimen are summarized in the following Table 2.

**Table 2**

| | Mean (Standard Deviation), n=14 | | | | |
|---|---|---|---|---|---|
| Regimen | Tₘₐₓ (hr) | Cₘₐₓ (µg/mL) | Cₘᵢₙ (µg/mL) | AUC₀₋₂₄ (µg·hr/mL) | DFL |
| A | 13.6 (6.3)* | 80.5 (18.6)* | 48.2 (17.0) | 1592 (402) | 0.523 (0.231) |
| B | 15.9 (4.5)* | 85.0(12.5)* | 55.1(13.3) | 1709 (276) | 0.432 (0.127)* |
| C | 3.6 (0.9) | 99.4 (15.7) | 54.1 (13.1) | 1789 (332) | 0.623 (0.160) |

| | | | | | |
|---|---|---|---|---|---|
| * Statistically significantly different from Regimen C. Regimen A: Divalproex Sodium ER; 2x500 mg once daily, fasting. Regimen B: Divalproex Sodium ER; 2x500 mg once daily, nonfasting. Regimen C: Depakote Tablet; 500 mg twice daily, fasting. | | | | | |

The mean Tₘₐₓ for Regimens A and B were about three-fold longer than that of Regimen C. The differences in Tₘₐₓ between Regimens A and C and between B and C were statistically significant. Regimens A and B tended to have lower Cₘₐₓ than that of Regimen C, and these differences were statistically significant. The regimens did not differ statistically significantly with respect to Cₘᵢₙ. The mean DFL for both ER Regimens A and B was lower than that of the reference, and the difference between Regimen B and the reference was statistically significant.

The 95% confidence intervals for bioavailability of the ER regimens relative to the reference for Cₘₐₓ and Cₘᵢₙ are given below. The point estimate for the ratio of the central values for both Cₘₐₓ and Cₘᵢₙ for Regimen A, and Cₘₐₓ for Regimen B, were lower than 1.0. The point estimate of the ratio for Cₘᵢₙ for Regimen B was approximately unity.

**Table 3**

| Relative Bioavailability | | | | | |
|---|---|---|---|---|---|
| Regimen | | Cₘₐₓ | | Cₘᵢₙ | |
| Test | Reference | Point Estimate | 95% Confidence Interval | Point Estimate | 95% Confidence Interval |
| A | C | 0.811 | 0.742 - 0.887 | 0.847 | 0.672 - 1.067 |
| B | C | 0.861 | 0.788 - 0.941 | 1.026 | 0.814 - 1.293 |
| Regimen A: Divalproex Sodium ER; 2x500 mg once daily, fasting. | | | | | |
| Regimen B: Divalproex Sodium ER; 2x500 mg once daily, nonfasting. | | | | | |
| Regimen C: Depakote Tablet; 500 mg twice daily, fasting. | | | | | |

The results for the two one-sided tests procedure for equivalence assessment of the regimens via a 90% confidence interval based on the natural logarithm of AUC₀₋₂₄ are given below.

**Table 4**

| Two One-Sided Tests Procedure for Equivalence Assessment, Day 6 AUC | | | |
|---|---|---|---|
| Relative Bioavailability | | | |
| Test | Reference | Point Estimate | 90% Confidence Interval |
| A | C | 0.891 | 0.817 - 0.971 |
| B | C | 0.970 | 0.890 - 1.058 |
| A | B | 0.918 | 0.842 - 1.001 |
| Regimen A: Divalproex Sodium ER; 2x500 mg once daily, fasting. | | | |
| Regimen B: Divalproex Sodium ER; 2x500 mg once daily, nonfasting. | | | |
| Regimen C: Depakote Tablet; 500 mg twice daily, fasting. | | | |

The 90% confidence intervals for AUC on Day 6 for the test ER composition administered under fasting (A) and nonfasting (B) conditions versus the reference fasting (C), both satisfied the 0.80 - 1.25 criterion for equivalence. Additionally, the 90% confidence interval for the ratio of central values of AUC for the test ER composition fasting:nonfasting regimens also satisfied the equivalence criterion.

The extended-release composition performs well. The extended-release regimens are equivalent to the reference regimen with respect to extent of absorption as characterized by AUC. The two test regimens did not differ statistically significantly from the reference regimen with respect to Cₘᵢₙ. The lower Cₘₐₓ and later Tₘₐₓ central values of the extended-release regimens compared the reference regimen suggest that the ER composition provides extended release of valproic acid *in vivo* under fasting and nonfasting conditions. The mean DFL for the extended-release composition administered under nonfasting conditions is lower (∼31%) than that of the reference regimen (observed means of 0.432 and 0.623, p<0.05). The mean DFL for the extended-release composition administered under fasting conditions was also lower (∼16%) than that of the reference regimen although statistical significance was not attained (observed means of 0.523 and 0.623, p=0.126).

### Example 3

### Multiple Dose Study

The bioavailability and plasma concentration-time profile of valproic acid from a new oral extended-release tablet composition of divalproex sodium (invention, made as in Example 1) was compared to that from the currently marketed divalproex sodium enteric-coated delayed-release tablet (Depakote® Abbott Laboratories; reference) under multiple-dose conditions.

Sixteen subjects enrolled in the study. They had a mean age of 34 years (range 19 - 55 years), mean height of 69 inches (range 65 - 75 inches), and mean weight of 180 pounds (range 148 - 209 pounds). This was a multiple-dose, open-label, 2-period, crossover study with no washout between periods in healthy adult male and female subjects comparing the extended-release (ER/invention) test composition (2 x 500 mg qd) with the delayed-release (DR/bid/prior art) Depakote enteric-coated tablet (500 mg q12h) as the reference. In one part of the study (Groups I and II), 4 subjects started on the ER test tablet in the morning and switched over to the 500 mg DR tablet bid on Day 7 (end of Period 1) and continued on it through Day 12 (Period 2). The other four subjects (Group II) started with the DR tablet and switched over to the ER test tablet in the morning of Day 7 and continued through Day 12. The second part of the study (Groups III and IV) was a repeat of the first part except that the test composition was given in the evening instead of in the morning. The ER composition was administered after a meal, and the DR tablet was given under fasting conditions.

A schematic of the compositions administered and the meal times follows.

**Table 5**

| Composition | Time of Dose | Breakfast | Lunch | Dinner | Snack |
|---|---|---|---|---|---|
| Morning Dose for ER Compositions | | | | | |
| ER | 6 am | 5:30 am | 12 N | 5:30 pm | 10:30 pm |
| DR | 6 am, 6 pm | 8:00 am | 12 N | 8:00 pm | 10:30 pm |

| Evening Dose for ER Compositions | | | | | |
|---|---|---|---|---|---|
| ER | 6 pm | 5:30 am | 12 N | 5:30 pm | 10:30 pm |
| DR | 6 pm, 6 am | 8:00 am | 12 N | 8:00 pm | 10:30 pm |
| ER = Extended-Release (invention); DR = Delayed-Release (prior art). | | | | | |

**Regimens:** The regimens administered were as follows.
A: Divalproex sodium extended-release tablets, 500 mg valproic acid equivalents; 2 x 500 mg tablets once every 24 hours starting with a morning dose. (invention)
B: Divalproex sodium enteric-coated delayed-release tablets (same as Depakote, Abbott Laboratories, reference); one 500 mg tablet once every 12 hours starting with a morning dose.
C: Divalproex sodium extended-release tablets, 500 mg valproic acid equivalents; 2 x 500 mg tablets once every 24 hours starting with an evening dose. (invention)
D: Divalproex sodium enteric-coated delayed-release tablets (same as Depakote, Abbott Laboratories, reference; one 500 mg tablet once every 12 hours starting with an evening dose.

Blood samples (7 mL) were taken at o, 12, 24, 36, 48, 60, 72, 84, 96, 108, 120, 121, 122, 123, 124.5, 126, 127.5, 129, 130.5, 132, 133, 134, 135, 136.5, 138, 139.5, 141, 142.5 and 144 hours from the first dose of each period. Blood samples were taken on the same schedule for Groups III and IV except that they were 12 hours later than for Groups I and II (i.e., first blood sample at 6 p.m. instead of 6 a.m.). Plasma samples were analyzed for valproic acid using a validated gas-liquid chromatographic method with flame ionization detection at Oneida Laboratories, New York.

### Pharmacokinetic and Statistical Analyses

Pharmacokinetic parameters were estimated by noncompartmental techniques. For Day 6 and 12 data, Cₘₐₓ, Tₘₐₓ, Cₘᵢₙ, AUC₀₋₂₄ and DFL were calculated. If Tₘₐₓ occurred after the second dose of Day 6 or 12, Tₘₐₓ was taken to be the time since the second dose rather than the time from the first dose.

Analyses of variance (ANOVAs) were performed for Tₘₐₓ, DFL, and for the natural logarithms of Cₘᵢₙ, Cₘₐₓ, and AUC₀₋₂₄. The model had effects for time (whether subject received ER composition in morning or evening), composition sequence, subjects nested within time by composition sequence, composition period, and the interaction of time with each of composition sequence, composition and period. Subject effects were random and all other effects were fixed. Equivalence of the two compositions with respect to AUC was addressed by performing the two one-sided tests procedure within the framework of the ANOVA on the logarithm of AUC. This confidence interval for relative bioavailability was obtained by exponentiating the endpoints of a 90% confidence interval for the difference of logarithm means (difference of composition main effects). As a further aid for assessing the characteristics of the ER composition, 95% confidence intervals for bioavailability relative to that of the reference composition were obtained from the ANOVAs for logarithms of Cₘᵢₙ and Cₘₐₓ.

The mean plasma valproic acid concentrations following administration of the 1000 mg test composition once every 24 hours (Regimens A and C) or the 500 mg reference composition once every 12 hours (Regimens B and D) for Days 6 and 12 are shown in Figure 4.

The pharmacokinetic results for Day 6 of each regimen are summarized in the following table.

**Table 6**

| Regimen | | Mean (% Coefficient of Variation) | | | |
|---|---|---|---|---|---|
| | | Cₘₐₓ (µg/mL) | Cₘᵢₙ (µg/mL) | AUC₀₋₂₄ (µg·hr/mL) | DFL |
| ER composition in morning (n=8) | | | | | |
| A | 0-24 hr | 87 (17.3) | 55.5 (38.7) | 1771 (22.8) | 0.46 (55.9) |
| B | 0-24 hr | 102 (10.5) | 53.3 (26.2) | 1798 (16.6) | 0.67 (31.2) |

| ER composition in evening (n=8) | | | | | |
|---|---|---|---|---|---|
| C | 0-24 hr | 85 (10.0) | 57.4 (14.9) | 1728 (12.5) | 0.39(19.7) |
| D | 0-24 hr | 98 (10.2) | 54.7 (13.9) | 1747 (10.5) | 0.60 (12.3) |

| All groups combined | | | | | |
|---|---|---|---|---|---|
| A and C | 0-24 hr | 86 (13.8) | 56.4 (28.1) | 1749 (17.9) | 0.42 (44.3) |
| B and D | 0-24 hr | 100 (10.3) | 54.0 (20.2) | 1773 (13.6) | 0.64 (24.8) |
| Regimen A: Divalproex Sodium ER; 2x500 mg in a.m., nonfasting. | | | | | |
| Regimen B: Depakote DR Tablet; 500 mg in a.m. and 500 mg in p.m., fasting. | | | | | |
| Regimen C: Divalproex Sodium ER; 2x500 mg in p.m., nonfasting. | | | | | |
| Regimen D: Depakote DR Tablet; 500 mg in p.m. and 500 mg in a.m., fasting. | | | | | |

There were no statistically significant differences in the pharmacokinetic results between subjects who received the ER composition in the morning and those who received the ER composition in the evening. Hence, the conclusions are based on the combined data of the groups.

The mean DFL of the ER composition was statistically significantly lower than that of the reference. The two compositions differed statistically significantly with respect to Cₘₐₓ, but not with respect to Cₘᵢₙ and AUC. For Cₘₐₓ and Cₘᵢₙ, the 95% confidence interval for bioavailability of the ER composition relative to that of the reference was 0.80 to 0.91 and 0.89 to 1.18, respectively. The 90% confidence interval by which the two one-sided tests procedure was performed for AUC was 0.924 to 1.041, being entirely within the equivalence range of 0.80 to 1.25.

Mean Cₘₐₓ for the test composition on Day 6 for both periods, when the plasma valproic acid concentrations were characterized, was lower than the reference composition and was statistically significantly different. Mean AUC₀₋₂₄ for Day 6 of each period was not significantly different between the test and reference compositions. Relative bioavailability based on the ratio (test: reference) of mean logarithm of AUC₀₋₂₄ (90% confidence interval) was 0.981 (0.924 to 1.041). The degree of fluctuation was statistically significantly smaller for the test composition (0.42) than for the reference (0.64). The results demonstrate the extended-release characteristics of the test composition and its similarity in bioavailability based on AUC when compared to the reference composition.

### Example 4

Based on the results of one multicenter, randomized, double-blind, placebo-controlled clinical trial, the composition of Example 1 (hereinafter "Depakote ER") was well tolerated in the prophylactic treatment of migraine headache. Of the 122 patients exposed to Depakote ER in the placebo-controlled study, 8% discontinued for adverse events, compared to 9% for the 115 placebo patients.

### a) Invention

The study below describes the side effect profile of a qd divalproex sodium dosage form according to this invention.

Table 11 includes those adverse events reported for patients in the placebo-controlled trial where the incidence rate in the Depakote ER-treated group was greater than 5% and was greater than that for placebo patients.

**Table 7**

| **Adverse Events Reported by >5% of Depakote Extended Release (ER/Invention) Patients During the Migraine Placebo-Extended Trial with a Greater Incidence than Patients Taking Placebo**^{**1**} | | |
|---|---|---|
| **Body System Event** | **Depakote ER (N=122)** | **Placebo (N=115)** |
| **Gastrointestinal System** | | |
| Nausea | 15% | 9% |
| Dyspepsia | 7% | 4% |
| Diarrhea | 7% | 3% |
| Vomiting | 7% | 2% |
| Abdominal Pain | 7% | 5% |

| **Nervous System** | | |
|---|---|---|
| Somnolence | 7% | 2% |

| **Other** | | |
|---|---|---|
| Infection | 15% | 14% |

| | | |
|---|---|---|
| ¹ The following adverse events occurred in greater than 5% of Depakote ER-treated patients and at a greater incidence for placebo than for Depakote ER: asthenia and flu syndrome. | | |

The following additional adverse events were reported by greater than 1% but not more than 5% of Depakote ER-treated patients and with a greater incidence than placebo in the placebo-controlled clinical trial for migraine prophylaxis:
Body as a Whole: Accidental injury, viral infection.
Digestive System: Increased appetite, tooth disorder.
Metabolic and Nutritional Disorders: Edema, weight gain.
Nervous System: Abnormal gait, dizziness, hypertonia, insomnia, nervousness, tremor, vertigo.
Respiratory System: Pharyngitis, rhinitis.
Skin and Appendages: Rash.
Special Senses: Tinnitus.

### b) Prior Art

The study below describes the side effect profile of Depakote DR.

Based on two placebo-controlled clinical trials and their long term extension, Depakote DR tablets were generally well tolerated with most adverse events rated as mild to moderate in severity. Of the 202 patients exposed to Depakote DR tablets in the placebo-controlled trials, 17% discontinued for intolerance. This is compared to a rate of 5% for the 81 placebo patients. The adverse events reported as the primary reason for discontinuation by greater than or equal to 1% of 248 Depakote DR-treated patients were alopecia (6%), nausea and/or vomiting (5%), weight gain (2%), tremor (2%), somnolence (1%), elevated SGOT and/or SGPT (1%), and depression (1%).

Table 8 includes those adverse events reported for patients in the placebo-controlled trials where the incidence rate in the Depakote DR-treated group was greater than 5% and was greater than that for placebo patients.

**Table 8**

| **Adverse Events Reported by >5% of Depakote Delayed Release (DR/prior art) Patients During Migraine Placebo-Extended Trials with a Greater Incidence than Patients Taking Placebo**^{**1**} | | |
|---|---|---|
| **Body System Event** | **Depakote DR (N=202)** | **Placebo (N=81)** |
| **Gastrointestinal System** | | |
| Nausea | 31% | 10% |
| Dyspepsia | 13% | 9% |
| Diarrhea | 12% | 7% |
| Vomiting | 11% | 1% |
| Abdominal Pain | 9% | 4% |
| Increased Appetite | 6% | 4% |

| **Nervous System** | | |
|---|---|---|
| Asthenia | 20% | 9% |
| Somnolence | 17% | 5% |
| Dizziness | 12% | 6% |
| Tremor | 9% | 0% |

| **Other** | | |
|---|---|---|
| Weight Gain | 8% | 2% |
| Back Pain | 8% | 6% |
| Alopecia | 7% | 1% |

| | | |
|---|---|---|
| ¹The following adverse events occurred in greater than 5% of Depakote DR-treated patients and at a greater incidence for placebo than for Depakote DR: flu syndrome and pharyngitis. | | |

The following additional adverse events not referred to above were reported by greater than 1% but not more than 5% of Depakote DR-treated patients and with a greater incidence than placebo in the placebo-controlled clinical trials:
Body as a Whole: Chestpain.
Cardiovascular System: Vasodilatation.
Digestive System: Constipation, dry mouth, flatulence, stomatitis.
Hemic and Lymphatic System: Ecchymosis.
Metabolic and Nutritional Disorders: Peripheral edema.
Musculoskeletal System: Leg cramps.
Nervous System: Abnormal dreams, confusion, paresthesia, speech disorder, thinking abnormalities.
Respiratory System: Dyspnea, sinusitis.
Skin and Appendages: Pruritus.
Urogenital System: Metrorrhagia.

Although the safety of ER and DR compositions were not assessed in the same study, a cross-study comparison of the data presented in Tables 11 and 12 suggest that the rate of adverse events were similar in the placebo-treated patients of the three well-controlled randomized studies. It is evident from Tables 11 and 12 that while the adverse events in the placebo-treated subjects were similar, Depakote ER-treated patients had lower number of adverse events compared to the Depakote DR-treated patients. It can be deduced that the reduced adverse events seen with Depakote ER treatment compared to Depakote DR treatment is probably due to the expected lower maximal plasma concentrations (Cₘₐₓ) and DFL that would be achieved, as illustrated in Examples 3 & 4, following administration of equal doses of two the compositions. It is reasonably believed that the reduced adverse effects, as well as lower frequency of dosing (once-a-day) dosing achieved with Depakote ER, would lead to better compliance.

The controlled release tablet compositions of the present invention thus provide an effective delivery system for the once daily administration of valproic acid (divalproex sodium) to patients in need of such treatment. The compositions of the invention provide substantially level plasma concentrations of valproic acid falling within the therapeutic range of the drug over a period which permits administration once daily. Further the incidence of side effects associated with valproate therapy has been reduced with this new composition.

While there have been shown and described what are the preferred embodiments of the invention, one skilled in the pharmaceutical composition art will appreciate that various modifications in the compositions and process can be made without departing from the scope of the invention as it is defined by the appended claims.

## Claims

1. Use of divaloproex sodium in the manufacture of a medicament for the treatment of migraine or bipolar disorder, the medicament comprising an oral controlled release composition suitable for once-a-day administration said composition on oral ingestion producing:
i) a Cₘₐₓ that is statistically significantly(p<0.05) lower than the Cₘₐₓ produced by an equal total daily dose of delayed release divalproex sodium tablet, when each is determined at steady state in a fasting population,
ii) a Cₘᵢₙ that is not statistically significantly (p<0.05) higher than the Cₘᵢₙ produced by said equal total daily dose of delayed release divalproex sodium tablet, when each Cₘᵢₙ is determined at steady state in a fasting population,
iii) an AUC value that is equivalent to the AUC value generated by said equal total daily dose of divalproex sodium delayed release tablet, when each AUC is determined at steady state in a fasting population,
in which said delayed release divalproex sodium has a tablet core comprising, (w/w):
| | |
|---|---|
| Divalproex sodium | 62.7% |
| Polyvinylpyrrolidone | 5.8% |
| Pregelatinised starch | 11.7% |
| Silicon dioxide | 19.8% |
and a coating comprising (%of core tablet weight)
| (a) subcoat | |
|---|---|
| Polyvinylpyrrolidone | 1.9% |
| Talc | 3.2% |
| Titanium dioxide | 0.6% |
| (b) enteric coat | |
|---|---|
| Hypromellose phthalate | 5% |
| Diacetylated monoglycerides | 0.5% |
| Dyes | 0.033% |
| Titanium dioxide | 0.35%. |

2. A use according to claim 1 in which the composition produces a DFL that is lower than the DFL produced by said delayed release divalproex sodium tablet, when each is determined at steady state in a fasting population.

3. A use to Claim 1 or Claim 2, in which the composition is a matrix system, an osmotic pump system or a reservoir polymeric system.

4. A use according to any one of the preceding claims in which said composition produces steady state peak plasma valproate levels that are about 10 to about 20% lower than that produced by a said delayed release divalproex sodium tablet.

## Patentansprüche

1. Verwendung von Divalproex-Natrium in der Herstellung eines Medikaments für die Behandlung von Migräne oder einer bipolaren Erkrankung, wobei das Medikament eine orale Zusammensetzung mit kontrollierter Freisetzung umfasst, die geeignet ist für die Verabreichung einmal am Tag, wobei die Zusammensetzung nach oraler Aufnahme folgendes erzeugt:
i) eine Cₘₐₓ, die statistisch signifikant (p<0,05) niedriger ist als die Cₘₐₓ, welche durch eine gleiche tägliche Gesamt-Dosis einer Divalproex-Natrium Tablette mit verzögerter Freisetzung erzeugt wird, wenn sie jeweils bei einem Fließgleichgewicht (steady state) in einer nüchternen Population bestimmt wird,
ii) eine Cₘᵢₙ, die nicht statistisch signifikant (p<0,05) höher ist als die Cₘᵢₙ, welche durch die gleiche tägliche Gesamt-Dosis einer Divalproex-Natrium Tablette mit verzögerter Freisetzung erzeugt wird, wenn die Cₘᵢₙ jeweils bei einem Fließgleichgewicht (steady state) in einer nüchternen Population bestimmt wird,
iii) einen AUC-Wert, der gleich dem AUC-Wert ist, der durch die gleiche tägliche Gesamt-Dosis einer Divalproex-Natrium Tablette mit verzögerter Freisetzung erzeugt wird, wenn die AUC jeweils bei einem Fließgleichgewicht (steady state) in einer nüchternen Population bestimmt wird,
worin das Divalproex-Natrium mit verzögerter Freisetzung einen Tablettenkern hat, der folgendes umfasst (m/m):
| | |
|---|---|
| Divalproex-Natrium | 62,7% |
| polyvinylpyrrolidon | 5,8% |
| Voc-verkleisterte Stärke | 11,7% |
| Siliziumdioxid | 19,8% |
und einen Überzug, der folgendes umfasst (% des Tablettenkerngewichts)
| (a) Unter-Überzug | |
|---|---|
| Polyvinylpyrrolidon | 1,9% |
| Talkum | 3,2% |
| Titandioxid | 0,6% |
| (b) Magensaftresistenter Überzug | |
|---|---|
| Hypromellosephthalat | 5% |
| Diacetylierte Monoglyceride | 0,5% |
| Farbstoffe | 0,033% |
| Titandioxid | 0,35% |

2. Eine Verwendung gemäß Anspruch 1, in welcher die Zusammensetzung einen DFL erzeugt, der niedriger ist als der DFL, der durch die Divalproex-Natrium Tablette mit verzögerter Freisetzung erzeugt wird, wenn er jeweils bei einem Fließgleichgewicht (steady state) in einer nüchternen Population bestimmt wird.

3. Eine Verwendung gemäß Anspruch 1 oder Anspruch 2, worin die Zusammensetzung ein Matrixsystem, ein osmotisches Pumpensystem oder ein polymeres Reservoirsystem ist.

4. Eine Verwendung gemäß irgendeinem der vorhergehenden Ansprüche, worin die Zusammensetzung Fließgleichgewichts(steady state) Peak Plasma Valproat-Werte erzeugt, die ungefähr 10 bis ungefähr 20% niedriger sind als die, die durch die Divalproex-Natrium Tablette mit verzögerter Freisetzung erzeugt werden.

## Revendications

1. Utilisation de divaloproex sodique pour la fabrication d'un médicament pour le traitement d'une migraine ou d'un trouble bipolaire, le médicament comprenant une composition orale à libération contrôlée appropriée pour une administration une fois par jour, ladite composition lors d'une ingestion orale produisant :
i) une Cₘₐₓ qui est statistiquement sensiblement inférieure (p < 0,05) à la Cₘₐₓ produite par une dose égale totale quotidienne de comprimé de divaloproex sodique à libération retardée, lorsqu'elles sont chacune déterminée à un état stable dans une population à jeun,
ii) une Cₘᵢₙ qui n'est pas statistiquement sensiblement supérieure (p < 0,05) à la Cₘᵢₙ produite par ladite dose égale totale quotidienne de comprimé de divaloproex sodique à libération retardée, lorsque chaque Cₘᵢₙ est déterminée à un état stable dans une population à jeun,
iii) une valeur de l'aire sous la courbe (AUC : area under ther curve) qui est équivalente à la valeur de l'aire sous la courbe générée par ladite dose égale totale quotidienne de comprimé de divaloproex sodique à libération retardée, lorsque chaque aire sous la courbe est déterminée à un état stable dans une population à jeun,
dans laquelle ledit divaloproex sodique à libération retardée a un noyau de comprimé (m/m) :
| | |
|---|---|
| divaloproex sodique | 62,7 % |
| polyvinylpyrrolidone | 5,8 % |
| amidon prégélatiné | 11,7 % |
| dioxyde de silicium | 19,8 % |
et un revêtement comprenant (% en poids du noyau du comprimé)
| (a) un sous-revêtement | |
|---|---|
| polyvinylpyrrolidone | 1,9 % |
| talc | 3,2 % |
| dioxyde de titane | 0,6 % |
| (b) un revêtement entérique | |
|---|---|
| phtalate d'hypromellose | 5 % |
| monoglycérides diacétylés | 0,5 % |
| colorants | 0,033 % |
| dioxyde de titane | 0,35 %. |

2. Utilisation selon la revendication 1, dans laquelle la composition produit un DFL qui est inférieur au DFL produit par ledit comprimé de divaloproex sodique à libération retardée, lorsque chaque DFL est déterminé à un état stable dans une population à jeun.

3. Utilisation selon la revendication 1 ou 2, dans laquelle la composition est un système matriciel, un système de type pompe osmotique ou un système polymérique de type réservoir.

4. Utilisation selon l'une quelconque des revendications précédentes, dans laquelle ladite composition produit des concentrations de valproate plasmatique du pic à l'état stable qui sont comprises dans la plage allant d'environ 10 à environ 20 % inférieurs à celles produites par ledit comprimé de divaloproex sodique à libération retardée.
